# EUROPEAN PATENT SPECIFICATION

(11) **EP 1 023 069 B1**
(45) Date of publication and mention of the grant of the patent: **05.11.2003**
(21) Application number: 98945685.0
(22) Date of filing: 15.09.1998
(51) Int. Cl.: A61K 31/465, A61K 9/00, A61K 9/70

(54) **NICOTINE COMPOSITIONS AND METHODS OF FORMULATION THEREOF**
NICOTIN ENTHALTENDE ZUSAMMENSETZUNGEN UND VERFAHREN ZUR IHRER FORMULIERUNG
COMPOSITIONS DE NICOTINE ET LEUR PROCEDE DE FORMULATION

(30) Priority: 25.09.1997 SE 9703458
(43) Date of publication of application: 02.08.2000
(73) Proprietor: Pharmacia Aktiebolag, 112 87 Stockholm (SE)
(72) Inventor: ANDERSSON, Sven, Börje, S-260 35 Öd kra (SE); JONN, Stefan, S-254 53 Helsingborg (SE); LANDH, Tomas, S-226 49 Lund (SE)
(74) Representative: Hedenström, John Allan
(86) International application number: SE9801632
(87) International publication number: WO99015171

(56) References cited:
- EP-A2- 0 289 342
- WO-A1-90/09808
- WO-A1-97/13528
- GB-A- 2 230 439
- US-A- 5 371 109
- US-A- 5 531 925
- DIALOG INFORMATION SERVICES, File 351, Dialog Accession No. 010450211, WPI Accession No. 95-351528/199546, KHALIL A.H. et al., "Lozenges Used to Treat Smoking Habit - Comprise Mixt. of Nicotine Resin and Substrate, Local Anaesthetic, Bulking Agent, Minerals, Vitamin(s) and Sweeteners"; & AU,B,662 877 (KHALIL A.H.) 14-09-95.
- INTERNATIONAL JOURNAL OF PHARMACEUTICALS, Volume 157, 1997, M.G. CARR et al., "Drug Delivery from a Liquid Crystalline Base Across Visking and Human Stratum Corneum", pages 35-42.
- DIALOG INFORMATION SERVICE, File 155, Medline, Dialog Accession No. 09208524, Medline Accession No. 95396883, WILSON A.L. et al., "Nicotine Patches in Alzheimer's Disease: Pilot Study on Learning, Memory and Safety"; & PHARMACOL. BIOCHEM. BEHAV., (UNITED STATES), Jun.-Jul. 1995, 51(2-3), p. 509-514.
- DIALOG INFORMATION SERVICE, File 155, Medline, Dialog Accession No. 08126991, Medline Accession No. 95167070, FAGERSTROM K.O. et al., "Nicotine May Relieve Symptoms of Parkinson's Disease"; & PSYCHOPHARMACOLOGY, (BERL.) (GERMANY), Sep. 1994, 116(1), p. 117-119.
- DIALOG INFORMATION SERVICE, File 155, Medline, Dialog Accession No. 09244966, Medline Accession No. 97001500, GUSLAND M. et al., "Pilot Trial of Nicotine Patches as an Alternative to Corticosteroids in Ulcerative Colitis"; & J. GASTROENTEROL, (JAPAN), Aug. 1996, 31(4), p. 627-629.

## Description

### Field of the invention

This invention relates to compositions comprising nicotine and methods to prepare said compositions useful in drug therapy, preferably tobacco substitution or replacement of tobacco and smoking cessation.

### Prior art

Nicotine replacement therapy as a smoking cessation strategy has been successful in the past. Previous nicotine-containing compositions aimed towards the purpose of reducing nicotine craving for subjects wishing to stop their use of tobacco products include e.g., US 3,845,217 disclosing chewable compositions, US 4,579,858 disclosing high-viscous nicotine nose-drop compositions, AU 664 415 disclosing low-viscous nicotine-containing compositions suitable for nasal spray administration, US 4,920,989 and 4,953,572 disclosing the use of inhalation aerosol, BP 1,528,391 and BP 2,030,862 disclosing liquid aerosol formulations adapted as mouth sprays, and for transdermal delivery of nicotine and US 4,915,950 disclosing the manufacturing of devices for transdermal delivery of nicotine. Several products based on the above mentioned patents are now marketed on an international scale.

A well known side-effect of nicotine is related to its concentration dependent local irritation. This adverse effect is particularly noticeable when nicotine formulations are applied topically, including the transmucosal, also comprising buccal and nasal, and transdermal administration routes. The concentration of nicotine in several of the above mentioned inventions, and product designs thereof, is hence limited by adverse effects caused by or related to its local irritation. There are, however, subjects which may have cravings for higher doses of nicotine than acceptable in applications of prior art administration forms. Furthermore, nicotine chewing gum formulations may cause unpleasant side-effects, besides local irritation, such as indigestion and nausea. As to the former increased transmucosal bioavailability decreases indigestion and hence local irritation caused by nicotine in the oral region. Also nicotine nasal spray formulations described in the aforesaid patent application is causing severe local irritation besides sneezing and tearing of the eyes.

EP 126,751 B1 discloses controlled release compositions for the delivery of biologically active materials. According to the invention described therein, a biologically active material is provided in formulations of amphiphilic substances capable of forming liquid crystalline phases when placed in contact with a liquid to be constituting any part of the polar compartment of the then formed thermodynamically stable phase. The liquid crystalline phases utilised in the aforesaid invention are preferably, but not exclusively, the group of cubic and hexagonal, liquid crystalline phases. WO 95/26715 discloses bioadhesive compositions using fatty acid esters forming liquid crystals, preferably the group of cubic and hexagonal, liquid crystalline phases. US 5,371,109 discloses the use of an L2-phase composed of mixtures of mono- and triglycerides and polar solvent as a controlled composition for biological active materials. The use of fatty acids in such mixtures is, however, not disclosed. US 5,531,925 discloses methods of preparing particles, or precursors thereof, of the aforementioned liquid crystalline phases or precursors thereof of reversed/inversed type II structure. WO 97/13528 also discloses liquid crystalline phases. Although in the latter nicotine is mentioned in the description, within a long list of drugs, no compositions according to the examples may be formulated with nicotine.

While is briefly mentioned in some of the above patent documents nowhere is disclosed how to formulate a stable liquid crystalline phases comprising nicotine. Surprisingly has now been found that the combination of nicotine and fatty acids is promoting the formation of liquid crystals of polar lipids. Therefore it has now become possible to produce liquid crystals comprising nicotine suitable for controlled release applications.

### Summary of the invention

Compositions for the therapeutic delivery of nicotine are provided. The compositions are used for administration of nicotine. The compositions are, furthermore, applicable for, but not restricted to, nasal, buccal, pulmonary and transdermal routes of administration. Suitable, but not limiting, administration forms are nasal sprays, buccal sprays, chewing gums, tablets, lozenges, transdermal or buccal patches, nasal gels, transdermal or buccal gels, transdermal or buccal adhesives, or sprays or aerosols for administration to the lungs. As is clear from the below description a transdermal patch comprising the present invention may have a very high loading implicating that a useful transdermal patch may have a very small area thereby being useful for placing on less visible sites of a human body, such as behind the ear.

According to one aspect, the invention is directed to compositions comprising nicotine which as a total forms a liquid crystalline phase or a precursor thereof comprising at least one amphiphilic lipid in sufficient amounts to form said liquid crystalline phase. The dependent variables of which the formation of the liquid crystalline phase from a precursor formulation are preferably chosen from, but not restricted to, compositional changes, temperature, and pressure, or any combinations thereof as can be realised by those skilled in the art of phase diagrams involving the mentioned state variables. Compositional changes are preferably those occurring when said formulations are put in contact with a body fluid and are preferably chosen from, but not restricted to, changes in solvent activity and ion activity including pH changes, or a combination thereof. Said changes can also be obtained by adding extraneous matter administered e.g. through flushing with aqueous solution prior to or after administration of the precursor at its site of action. Liquid crystalline phase formations induced by temperature are preferably, but not exclusively, those formed by the increase in temperature caused by contact with a body, preferably, but not exclusively, a human body, when said formulation is put in contact with any site of application on a body which causes the formulation to adopt a higher temperature at which another phase is formed. Similar pressure induced phase transformations can be utilized in accordance with e.g. pressure-compositional phase diagrams known to or easily established by those skilled in the art.

Said precursor is preferably chosen from, but not restricted to, another liquid crystalline, a solid phase, a solution phase, or any other phase structure, which is capable of undergoing phase transition by means of any of the above-mentioned state variables so to transform to the desired liquid crystalline phases in question.

In yet another embodiment, the invention is directed to an article of compositions directed to the production of spontaneously forming dispersions of liquid crystalline phases in which said compositions nicotine, one fatty acid and one monoglyceride in proportions sufficient to form a liquid crystalline phase when put into contact with a polar solvent, preferably of, but not restricted to, aqueous nature, which upon further dilution with said polar solvent, or another polar solvent, undergoes transformation to a stable colloidal dispersion of the liquid crystalline phase or a precursor thereof.

According to another aspect, the invention is directed to formulation of liquid crystalline compositions or precursors thereof comprising at least one monoglyceride, at least one fatty acid, and nicotine in which nicotine and said fatty acid forms an ion-pair complex. Formulation of said liquid crystalline phase in e.g., a buffered aqueous environment, such as saliva or mucosa, causes the nicotine-ion pair complex to be weakened at a certain pH causing nicotine to be released in a controlled fashion from the liquid crystalline matrix, its precursor or its offspring.

The term "precursor" used herein and in the claims refers to any formulation or state of formulation of the present invention which by means of changing one or several state variables in such a way that one or a multiple of the liquid crystalline phase(s) containing nicotine forms *in situ* as disclosed herein.

The term "offspring" used herein and in the claims refers to any resulting state of the present invention resulting from changes in the state variables after application of the present invention.

The term "nicotine" as used herein and in the claims encompasses nicotine base and its mono- and dicationic species, nicotine resinate (e g according to US 3,845,217) and nicotine complexes. Nicotine salts preferably, but not exclusively, encompass nicotine hydrochloride, nicotine dihydrochloride, nicotine sulphate, nicotine monotartrate, nicotine bitartrate, nicotine zinc chloride and nicotine salicylate. Further the term "nicotine" also encompasses nicotine metabolites and nicotine type compounds, preferably, but not exclusively, cotinine, myosmine, anabasine, anatabine, nornicotine, beta-nicotyrine, beta-nornicotyrine and nicotine-N-oxides.

The term "liquid crystalline phase" used herein and in the claims has the meaning of a thermodynamically stable state of matter which lacks short range order, thus not true crystalline, but possesses long range order, thus not liquid or amorphous.

The term "cubic liquid crystalline phase" and "cubic phase" as used herein has the meaning of an isotropic liquid crystalline phase whose long range order is characterized by one of the possible cubic space group arrangements as determined by X-ray scattering methods in combination with phase diagram studies, said methods being known to those skilled in the art.

The term "hexagonal liquid crystalline phase" or "hexagonal phase" as is used herein denotes an anisotropic liquid crystalline phase whose long range order is characterized by its X-ray scattering pattern revealing a two-dimensional lattice.

The term "L2-phase" as used herein and in the claims denotes an optical isotropic solution phase characterized by the lack of both long and short range order and is used synonymously to the term microemulsion.

The meaning of the "type" i.e., type I or type II, or synonymously reversed (inversed) or normal, respectively, crystalline phase as used herein and in the claims denotes the curvature of the apolar-polar interface as defined by the direction of the normal to the interface as towards the polar or apolar constituents, respectively, following the terminology used in the current literature in the art. The type of phase is readily distinguished by the behaviour of the phase upon increasing amount of polar constituents e.g., water dilution, upon which type I phases will be diluted and eventually transformed to another phase e.g. normal micelles while phases of type II will swell to a certain point of water activity at which they will be in equilibrium with any further added polar constituents e.g. water.

The meaning of "adhesiveness" as used herein and in the appended claims denotes the capacity of a liquid crystalline phase so formulated as it has the capacity to change its state by e.g. absorption of polar constituents from the surroundings of its site of application, causing it to further swell, or causing a phase transformation. This is readily appreciated, by those skilled in the art, to be a consequence of the phase behaviour of the formulation in question in the environment of the site of application with which it is striving to approach uniformity in the sense of thermodynamics and mass transport which result in an adhesiveness through the driving force to reach close apposition caused by its thermodynamic degree of freedom followed by the establishment of non-covalent bonds to the surface or site of application.

The term "state variables" as used herein and in the claims denotes any of the alterable parameters defining the phase behaviour as expressed by Gibb's phase rule known to those skilled in the art.

The meaning of the term "thermodynamically stable" as used herein and in the appended claims denotes a physical composition whose stability with respect to the integrity of its structure is limited only by chemical degradation.

In the present application such anionic surfactants are preferred which are capable of forming ion pairs or salts so favouring the formation of liquid crystalline phases.

### Description of the figures

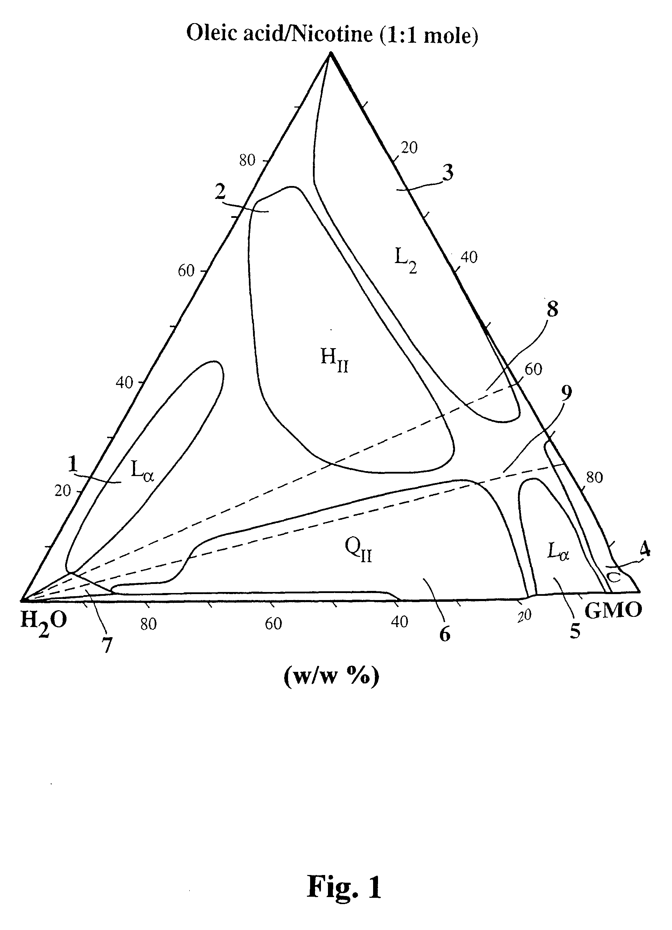

**Figure 1** shows a four component phase diagram oleic acid-nicotine (1:1 mole ratio)/monoolein/water illustrating various physical phases of oleic acid-nicotine (1:1 mole ratio)/monoolein/water mixtures which may be used for predicting embodiments of the invention. The phases indicated are: lamellar phase form 1 and 5, reversed hexagonal phase form 2, microemulsion of L₂ phase form 3, solid crystalline form 4, cubic phase form 6, and three-phase dispersion area 7. Phase determination by means of small angle X-ray and polarizing microscopy was performed according to Landh, T., J. Phys. Chem. 98, 8453 - 8467, 1994. Examples of phase progression upon the addition of aqueous solution are shown in 8 and 9.

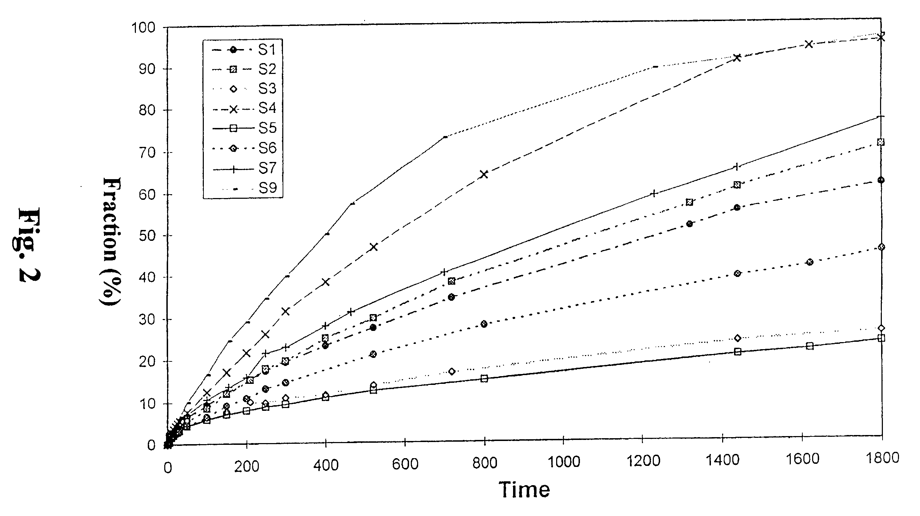

**Figure 2** shows *in vitro* over time (minutes) release of nicotine performed in USP paddle dissolution test. Compositions of samples are given in Table 1.

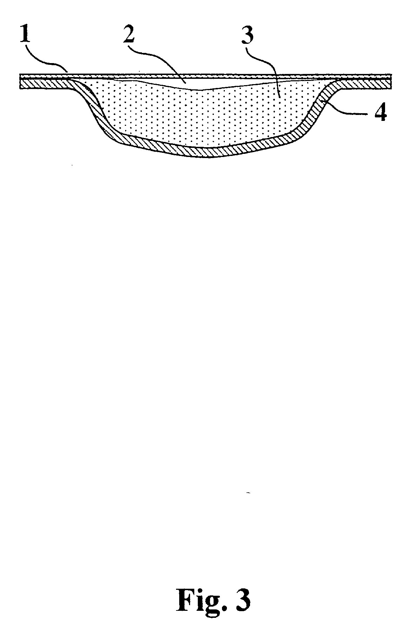

Figure 3 shows a buccal patch device with a release liner 1, an optional empty cavity 2, a controlled release matrix 3 of any of the liquid crystalline compositions mentioned herein and a backing 4.

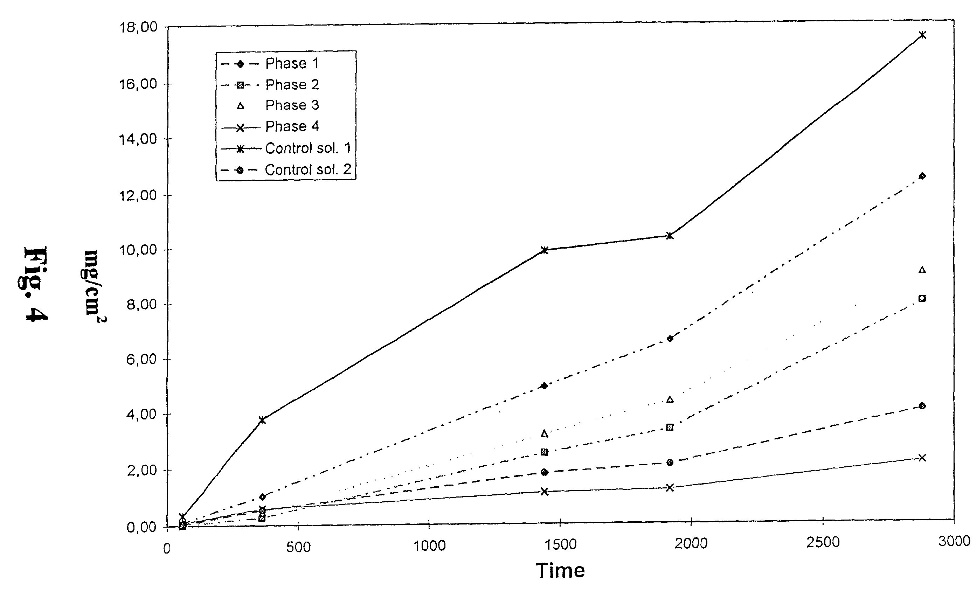

**Figure 4** shows *in vitro* skin (pig skin from the back) permeation over time (minutes) of nicotine from the liquid crystalline phases given in Table 2. The experiments were performed in a Franz diffusion cell with a available diffusion area of 1.8 cm². Experiments were performed at room temperature and with a receptor phase at 37 centigrades. About 180 mg of samples in Table 2 were applied to the skin. Two control solutions were employed - control sol. 1 and 2 respectively, consisting of 114 mg nicotine/g water (pH = 10.80) and 114 mg/g phosphate buffer (pH = 6.95).

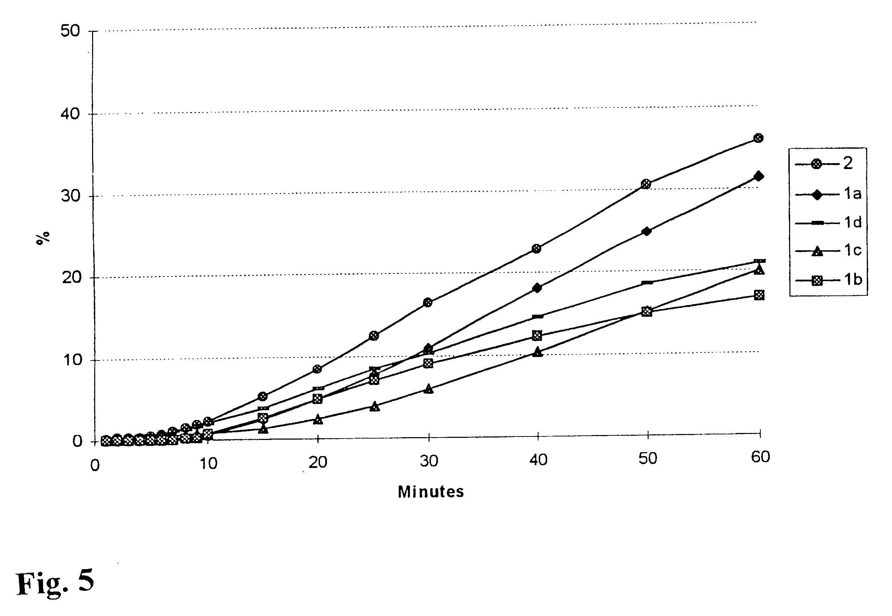

**Figure 5** shows examples of *in vitro* permeation of nicotine through pig buccal epithelium. Compositions of the applied liquid crystalline phases are given in Table 3. The experiments were performed in a Franz diffusion cell with a available diffusion area of 1.8 cm². Experiments were performed at room temperature and with a receptor phase at 37 centigrades. About 55 mg of samples in Table 3 were applied to the buccal epithelium corresponding to an amount of 1 mg nicotine per experiment. Experiments, conditions and average fluxes of permeated nicotine are given in Table 4.

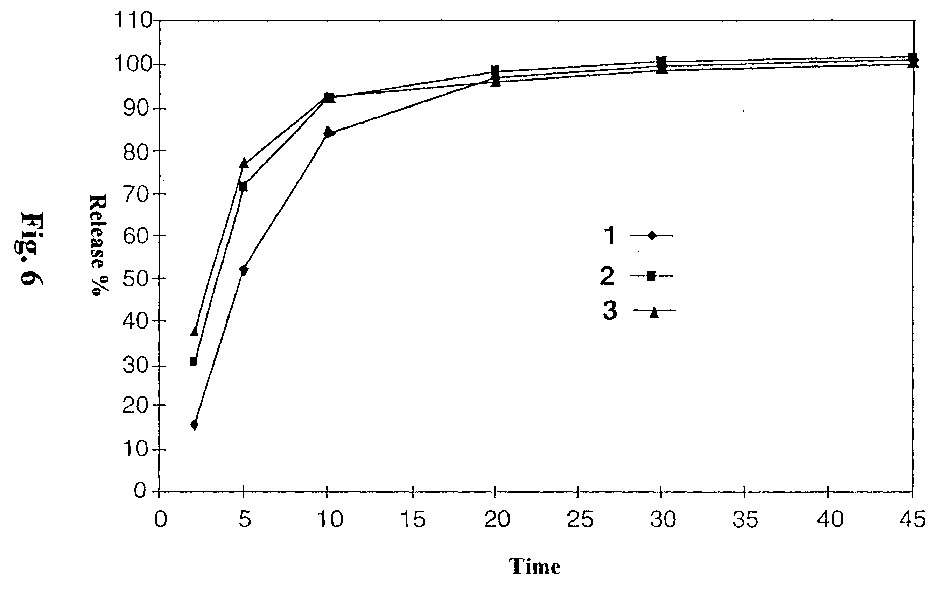

**Figure 6** shows *in vitro* release of nicotine over time (minutes) from liquid crystalline phase compositions formulated in a chewing gum base with the appropriate excipients known to those skilled in the art. Sample 1, shown with the line having a diamond symbol, corresponds to commercially available Nicorette® 2 mg as manufactured by Pharmacia & Upjohn.. Compositions of sample 2, shown with the line having a square symbol, and of sample 3, shown with the line having a triangle symbol, are given in Example 8. Experiments were essentially performed as described in US 5,087,424.

**Table 1**

| *Wt % of components* | | | | | | |
|---|---|---|---|---|---|---|
| **Sample No** | **Glycerol-monoolein** | **Oleic acid** | **Nicotine** | **Water** | **Buffer** **pH 5** | **Buffer** **pH 7** |
| S1:1 | 47.50 | 1.84 | 3.16 | 47.50 | | |
| S1:2 | 47.50 | 1.84 | 3.16 | 47.50 | | |
| S2:1 | 47.50 | 3.18 | 1.82 | 47.50 | | |
| S2:2 | 47.50 | 3.18 | 1.82 | 47.50 | | |
| S4:1 | 23.75 | 3.18 | 1.82 | 71.25 | | |
| S4:2 | 23.75 | 3.18 | 1.82 | 71.25 | | |
| S5:1 | 47.50 | 4.20 | 0.80 | 47.50 | | |
| S5:2 | 47.50 | 4.20 | 0.80 | 47.50 | | |
| S6:1 | 71.25 | 3.18 | 1.82 | 23.75 | | |
| S6:2 | 71.25 | 3.18 | 1.82 | 23.75 | | |
| S7:1 | 47.50 | 3.18 | 1.82 | | 47.50 | |
| S7:2 | 47.50 | 3.18 | 1.82 | | 47.50 | |
| S9:1 | 47.50 | 3.18 | 1.82 | | | 47.50 |
| S9:2 | 47.50 | 3.18 | 1.82 | | | 47.50 |

**Table 2**

| *Wt % of components* | | | | | | | |
|---|---|---|---|---|---|---|---|
| **Sample No** | **Glycerol-monoolein** | **Oleic acid** | **Nicotine** | **Benzyl alcohol** | **Glycerol** | **Water** | **Buffer** **pH 7** |
| 1 | 45.96 | 11.50 | 11.47 | | 11.56 | | 19.51 |
| 2 | 45.96 | 20.07 | 11.46 | | 11.56 | | 10.97 |
| 3 | 45.95 | 11.50 | 11.46 | | 11.55 | 19.54 | |
| 4 | 2.00 | 0.35 | 1.00 | 0.90 | | 95.75 | |

**Table 3**

| *Amount of components in gram*. | | | | | |
|---|---|---|---|---|---|
| **Sample No** | **Glycerol-monoolein** | **Oleic acid** | **Nicotine** | **Water** | **Buffer** **pH 7** |
| 1 | 4.7502 | 0.3182 | 0.1818 | 4.7484 | |
| 4 | 2.3759 | 0.3180 | 0.1818 | 7.1259 | |
| 6 | 7.1253 | 0.3184 | 0.1825 | 2.3741 | |
| 9 | 4.7503 | 0.3182 | 0.1817 | | 4.7501 |

**Table 4**

| *Average of fluxes of nicotine (µg*/*cm*^{*3*}/*h ± SD) (n=3)* (Experiment 1a, 1b, 1c, 1d and 2)* | | | | | |
|---|---|---|---|---|---|
| **Sample No** | **Experiment 1a** | **Experiment 1b** | **Experiment 1c** | **Experiment 1d** | **Experiment 2** |
| 1 | 132 ± 68 ** | 112 ± 41 | 165 ± 76 | 117 ± 55 | 257 ± 29 |
| 4 | 229 ± 75 | 89 ± 15 | 226 ± 0 ** | 134 ± 31 | 201 ± 49 |
| 6 | 166 ± 44 | 82 ± 31 | 223 ± 81 ** | 147 ± 41 | 160 ± 51 |
| 9 | 249 ± 89 | 134 ± 9 | 146 ± 53 | 143 ± 37 | 277 ± 101 |

| | | | | | |
|---|---|---|---|---|---|
| ^{*)} **1a:** Artificial saliva as donorphase, backing membrane on cubic phases. **1b:** Artificial saliva as donorphase, no backing membrane on cubic phases. **1c:** Buffer pH 7 as donorphase, backing membrane on cubic phases. **1d:** Buffer pH 7 as donorphase, no backing membrane on cubic phases. **2:** No donorphase, backing membrane on cubic phases. | | | | | |
| ^{**)} n=2 | | | | | |

### Detailed description of the invention

Compositions of the invention includes an active drug, preferably nicotine. More precisely, the compositions comprise nicotine formulated in liquid crystalline phases or any precursor or offspring thereof, and dispersions, preferably, but not restricted to, colloidal dispersions of said liquid crystalline phases, their precursors or offspring. Specifically the invention includes controlled release compositions directed to formulations of the biologically active compound, preferably, but not exclusively, nicotine, in type I and II cubic liquid crystalline phases, type I and II hexagonal liquid crystalline phases, type I and II intermediate liquid crystalline phases, and lamellar phases, in all cases irrespective of space group arrangement, and precursors of or offspring to said liquid crystalline phases, including any different phase or mixture thereof as adopted upon or during application of the formulation by the so induced change or changes taking place through changes of a physical and or a chemical nature having an effect on one or more of the state variables defining the system.

Precursor and offspring phases thus include, besides the above mentioned liquid crystalline phases, any phase which is not characterized as a liquid crystalline phase including, but not restricted to, solid phases, solution phases of micellar type I and II, solution phase of bilayered type including the sponge phase (L₃ phase), the L₂ phase, and true solutions.

Preferably compositions of the invention relates to the use of said liquid crystalline formulations, precursors thereof and offspring thereof in tobacco substitution, smoking cessation and smoking replacement. More specifically, said liquid crystalline formulations, precursors thereof and offspring thereof are useful in said therapies when applied alone, or in appropriate devices known to those skilled in the arts of drug delivery and dosage forms, transdermally and through mucosa. The aforementioned liquid crystalline formulations, precursors and offspring thereof can be applied to plasters, patches, chewing gum, lozenges or other devices used in combination or in conjunction with said liquid crystalline formulations, precursors and offspring thereof. More specifically, the aforesaid liquid crystalline formulations, precursors thereof and offspring to are preferably applied as, or in conjunction with, transdermal dosage forms of nicotine and buccal dosage forms of nicotine. Most preferably, said liquid crystalline formulations, precursors and offspring thereof are applicable to buccal delivery of nicotine in several dosage forms, including, but not restricted to, chewing gum, lozenges, sublingual tablets and patches, including dosage forms used as wet or chewable tobacco replacement. Aforementioned compositions of said liquid crystalline formulations, precursors and offspring thereof are applicable to coat a bulk carrier system of preferably, but not restricted to, inert nature. Most preferably, said coating is applied on starch granules or powder, or plastic or other polymeric material.

Formulations of the aforementioned dispersed liquid crystalline formulations, precursors and offspring thereof nicotine comprising a biologically active agent, preferably, but not exclusively, being nicotine, in which the liquid crystalline phase, its precursors or offspring are preferably selected from, but not restricted to, colloidal particles suitable for nasal spray or drop dosage forms and mouth spray or drop dosage forms. The dispersed liquid crystalline formulations, precursors and offspring thereof, are preferably selected from the group of, but not restricted to, type I and II cubic liquid crystalline phases, type I and II hexagonal liquid crystalline phases, type I and II intermediate liquid crystalline phases, and lamellar phases, in all cases irrespective of space group arrangement, and precursors of or offspring to said liquid crystalline phases, including any different phase or mixture thereof than adopted upon or during application of the formulation by the so induced change or changes taking place through changes of a physical and or a chemical nature having an effect on one or more of the state variables defining the system. Stable dispersed particles of the aforementioned liquid crystalline phase or mixture of phases or their precursors or offspring are readily produced by various fragmentation methods known to those skilled in the art. Preferable is spontaneous formation of stable colloidal dispersions of the aforementioned liquid crystalline phase or mixture of phases or their precursors. The aforementioned colloidal particulate formulations, containing nicotine, of the aforesaid liquid crystalline phases or precursors or offspring thereof can be applied to nasal and buccal/sublingual drop and spray dosage forms and pulmonary aerosol dosage form or other devices used in combination or in conjunction with said liquid crystalline formulations, precursors or offspring thereof. More specifically, the aforesaid liquid crystalline formulations, precursors and offspring thereof are preferably applied in, or in conjunction with, dosage forms intended for nasal, pulmonary or buccal administration of nicotine.

Specifically, compositions of the invention include one or more surface active agents, preferably, but not restricted to, one or more polar lipids, chosen from the non-limiting group of glycolipids, phospholipids, monoglycerides and diglycerides or a mixture thereof, preferably those polar lipids which are known, to those skilled in the art, to form liquid crystalline phases in equilibrium with any of the factors defined by a state variable.

More specifically, compositions of the invention include one or more biologically active agents, preferably nicotine, one or more monoglycerides preferably chosen from, but not restricted to, the group consisting of glycerol esters of palmitoleic acid, oleic acid, linoleic, linolenic and arachidonic acid. Most preferable is the glycerol ester of oleic acid. Optional components besides usual pharmaceutical excipients, include, but are not restricted to, one or more fatty acids preferably chosen from, but not restricted to, the group of stearic acid, palmitic acid, oleic acid, linoleic acid, linolenic acid, arachidonic acid, one or more polar solutions, such as aqueous solutions, glycerol or propyleneglycol, or a mixture thereof, one or more local analgesic(s), all components in relative amounts so as the formulation forms or is capable of forming a liquid crystalline phase by change of any of the state variables composition, temperature and pressure, or a combination thereof. In addition are included usually employed pharmaceutical excipients such as flavouring agents, sweeteners, buffering agents, preservatives and such components can be added without departing from the gist of the invention.

The embodiments, practice and methods of manufacturing the compositions of this invention is further illustrated by the following non-limiting examples.

### Example 1

Glycerol monooleate, oleic acid, nicotine, glycerol purum and water according to the following composition

| **Component:** | **Weight %:** |
|---|---|
| Glycerol monooleate | 45 |
| Oleic acid | 10 |
| Nicotine | 10 |
| Glycerol | 10 |
| Phosphate buffer (pH 7.0) | 15 |

are mixed at room temperature. The above example can be prepared in different ways. One way is as follows: to solid glycerol monooleate is added oleic acid and the mixture is allowed to form a solution to which nicotine is added. To the so obtained solution glycerol is added and the mixture is allowed to form a solution to which water is added to form a cubic liquid crystalline phase.

This composition of the invention in this application is useful in tobacco substitution, replacement and cessation therapies in a number of different ways. The composition is inserted as an adhesive gel applied directly to the buccal mucosa at which site nicotine is delivered through it. The composition is melted and poured into patch devices as illustrated in Figure 3 which is applied to a desired topical site of action such as the inner cheek at which site nicotine is released.

### Example 2

Compositions according to Table 1 were prepared in duplicates as described in Example 1 and the *in vitro* release of nicotine in phosphate buffer (pH 7.0) from the compositions was determined by means of commercially available instrument for testing dissolution according to USP. The results are shown in Figure 2.

It is readily appreciated that the composition controls the rate of release. Important factors are the nicotine:oleic acid ratio and the water content. It is thus shown that the present invention can be used to control the release rate of nicotine.

### Example 3

Glycerol monooleate, oleic acid, bensyl alcohol, nicotine, and water were mixed according to the following composition

| **Component:** | **Weight %:** |
|---|---|
| Glycerol monooleate | 8 |
| Oleic acid | 4 |
| Bensyl alcohol | 4 |
| Nicotine | 4 |
| Water | 80 |

The above example can be prepared in different ways. One way is as follows: to solid glycerol monooleate is added oleic acid and bensyl alcohol and the mixture is allowed to form a solution to which nicotine is added. To the so obtained solution water is added and the mixture is allowed to form a hexagonal liquid crystalline phase of type I.

This composition of the invention in this application is useful in tobacco substitution, replacement and cessation therapies in a number of different ways. The composition is inserted as an adhesive gel applied directly to the buccal mucosa at which site nicotine is delivered through it. The composition is melted and poured into patch devices as illustrated in Figure 3 which is applied to a desired topical site of action such as the inner cheek at which site nicotine is released.

### Example 4

Glycerol monooleate, oleic acid, benzocaine, and nicotine were mixed in the following proportions:

| **Component:** | **Weight %:** |
|---|---|
| Glycerol monooleate | 2 |
| Oleic acid | 1 |
| Benzocaine | 1 |
| Nicotine | 1 |
| Water | 95 |

The above example can be prepared in different ways. One way is as follows: to solid glycerol monooleate is added oleic acid and nicotine and the mixture is allowed to form a solution to which benzocaine is added and let to dissolve. To the so obtained solution one fifth of the total amount of water as indicated in the table is added and the mixture is allowed to form a hexagonal liquid crystalline phase of type I to which the remaining water is added upon which a stable colloidal dispersion is spontaneously formed. The composition of the invention of this application is applicable in tobacco substitution, replacement and cessation therapies in a number of different ways as exemplified in the following. The composition is dropable and sprayable using a standard device for nasal administration of nicotine. For similar purposes, it is useful as mouth drops or spray. Furthermore, it forms liquid aerosols using standard devices known to those skilled in the art with particle sizes in the range of the size of colloidal particles suited for pulmonary delivery of nicotine through the lung mucosa.

### Example 5

Bensyl alcohol and nicotine is mixed to form a solution to which water is added to reach a final composition of:

| **Component:** | **Weight %:** |
|---|---|
| Nicotine | 1 |
| Bensyl alcohol | 1 |
| Water | 98 |

The above example can be prepared in different ways and optional components such as preservatives, buffer, sweetness and flavouring agents can be added. The composition can be delivered to the nasal mucosa by means of a metered spray device such as described in US 4,579,858 for use in tobacco substitution, replacement and cessation therapies. Furthermore, the composition can be used similarly as a mouth spray for application of the solution directly to the oral region and its mucosal lining.

### Example 6

Compositions according to Table 2 were prepared as described in Example 1 above and tested for the *in vitro* skin permeation of nicotine. The results are shown in Figure 4 and the experimental design as described in the figure legend. It is readily appreciated that the current invention can be used for controlled skin permeation of nicotine.

### Example 7

Compositions according to Table 3 were prepared as described in Example 1 and tested for the *in vitro* pig buccal epithelium permeation of nicotine. The results are given in Figure 5 with average nicotine fluxes compiled in Table 4. The experimental design is given in the legend to Table 4. It is readily appreciated that the current invention provide controlled release of nicotine and controlled fluxes and permeation through pig buccal epithelium *in vitro*.

### Example 8

Compositions as below

| Component: | **Comp. 2** | **Comp. 3** |
|---|---|---|
| | **Weight %** | |
| Glycerol monooleate | 45.95 | 45.95 |
| Oleic acid | 11.50 | 20.02 |
| Nicotine | 11.46 | 11.46 |
| Glycerol | 11.55 | 11.55 |
| Buffer pH 7 | 19.54 | 11.02 |

can be mixed with a gum base and optional flavouring agents according to the following example

| **Component:** | **Weight %:** |
|---|---|
| Liquid crystalline phase composition according to comp. 2 or 3 above | 2 |
| Gum base (from Dreyco) | 77 |
| Sorbitol power | 15 |
| Sorbitol solution (70%) | 4 |
| Flavouring agents | 2 |

which with the appropriate techniques can be treated by conventional means to result in a chewing gum. The *in vitro* release of nicotine from the above chewing gum formulations determined according to US 5,087,424 is shown in Figure 6 together with that of Ni-corette ® 2 mg. It is readily appreciated by those skilled in the art that the composition controls the release rate of nicotine. Also the use of precursors to the herein disclosed liquid crystalline phases is readily appreciated in which case the change is brought out by change in state variables chosen from chemical composition, preferably of aqueous activity, or body temperature, or a combination thereof.

Further aspects of the invention can be appreciated from figure 1.

Figure 1 shows a triangular diagram used to illustrate the phase behaviour of a four component system consisting of oleic acid:nicotine, glycerol monooleate and water. The non-limiting example of phase behaviour shown in figure 1 represent 1:1 molar ratio of the oleic acid:nicotine mixture. Other ratios are applicable as well.

Shown in this diagram are some of the physical phases formed at various compositions represented by the diagram. These phases may be, for example, a water-rich one-dimensional lamellar liquid crystalline phase 1, a hexagonal type II liquid crystalline phase 2, a free-flowing liquid phase 3, a solid crystalline phase 4, a water-poor one-dimensional lamellar liquid crystalline phase 5 and a three-dimensional cubic liquid crystalline phase of type II 6.

It can be appreciated from the figure that a composition of phase 3 along the indicated line 8 will upon contact with water or any polar liquid such as saliva or any other body fluid changes effectively the physical state of the formulation towards the water comer along line 8. Thus by exposure to more water the free-fluid composition will increase its viscosity once it adopts the hexagonal liquid crystalline state of type II and subsequently enter more-phase areas (not shown in figure 1) to finally enter a three component phase area in which the cubic liquid crystalline phase coexist with the lamellar phase 1 according to the composition given along line 8. Within this three-phase area the cubic liquid crystalline phase 6 is easily dispersed and form small particles surrounded by the lamellar phase 1.

As known to those skilled in the art adhesiveness to such surfaces as the mucous lining of the mouth is obtained and significantly increased through the absorption of water and the successive changes of physical states such as appreciated along line 8 in figure 1.

Similar a solid crystalline phase 4 with a composition along line 9 goes through successive changes of physical states upon the uptake or addition of water as illustrated along line 9 to finally enter the aforementioned three-phase area illustrated along line 8.

Above has been disclosed utility of nicotine-containing liquid crystalline phases for tobacco substitution or replacement of tobacco and smoking cessation. Anyhow, for persons skilled in the art use of said phases is evident for treatment of other indications, such as Alzheimer's disease, Parkinson's disease and ulcerative colitis, for which it is known that nicotine has a curative effect.

## Claims

1. A composition comprising nicotine, one or more polar lipids and one or more anionic surfactants in sufficient amounts to form a liquid crystalline phase or a precursor or offspring thereof when placed in a polar solvent.

2. A composition according to claim 1 wherein the polar solvent is an aqueous solutions, glycerol or propyleneglycol, or a mixture thereof.

3. A composition according to anyone of claims 1 or 2 wherein the liquid crystalline phase or precursor or offspring thereof is anyone of the below types:
type I or type II cubic liquid crystalline phases,
type I or type II hexagonal liquid crystalline phases,
type I or type II intermediate liquid crystalline phases, and lamellar phases, in all cases irrespective of space group arrangement, and precursors of or offspring to said liquid crystalline phases, including any different phase or mixture thereof as adopted upon or during application of the formulation by the so induced change or changes taking place through changes of a physical and or a chemical nature having an effect on one or more of the state variables defining the system,
solid phases,
solution phases of micellar type I or type II,
solution phase of bilayered type including the sponge phase (L₃ phase), the L₂ phase, microemulsions, and true solutions.

4. A composition according to anyone of claims 1 - 3 wherein the one or more polar lipids are monoglycerides.

5. A composition according to anyone of claims 1 - 4 wherein the one or more anionic surfactants are fatty acids.

6. A composition according to claim 5 wherein the one or more fatty acid(s) is/are chosen from the group consisting of stearic acid, palmitic acid, oleic acid, linoleic acid, linolenic acid and arachidonic acid.

7. A composition according to claim 6 wherein the fatty acid is oleic acid.

8. A composition according to anyone of claims 1 - 7 further comprising a local analgesic or a mixture of local analgesics.

9. A composition according to claim 8 wherein the local analgesic(s) is/are selected from bensyl alcohol, benzocaine, chlorbutanol, chloroprocaine, clove, eugenol, lidocain, lidocain hydrochloride, mepivacaine, phenol, prilocaine, procaine, tetracaine, tetracaine hydrochloride and salicyl alcohol, or combinations thereof.

10. A composition according to claim 9 wherein the local analgesic is bensocaine.

11. A composition according to claim 9 wherein the local analgesic is bensyl alcohol.

12. A composition according to anyone of claims 3 - 11 wherein the one or more monoglyceride(s) is/are chosen from the group consisting of glycerol esters of palmitoleic acid, oleic acid, linoleic, linolenic and arachidonic acid.

13. A composition according to anyone of claims 3 - 11 wherein the monoglyceride is monoolein.

14. A composition according to anyone of claims 3 - 11 wherein the monoglyceride is monolinolein.

15. A composition according to anyone of the preceding claims, optionally further comprising pharmaceutically acceptable excipients, for use as a pharmaceutical.

16. A composition according to claim 15 wherein the optional pharmaceutically acceptable excipients are chosen from the group consisting of flavouring agents, sweeteners, buffering agents and preservatives.

17. A composition according to anyone of claims 15 or 16 for use in tobacco substitution, replacement of tobacco or smoking cessation.

18. A composition according to anyone of claims 15 or 16 for treating Alzheimer's disease, Parkinson's disease or ulcerative colitis.

19. A composition according to anyone of claims 15 - 18 for nasal, buccal, transdermal, mucosal or pulmonary administration.

20. A composition according to anyone of claims 15 - 18 for administration via a nasal spray or gel, a buccal spray, a chewing gum, a tablet, a lozenge, a transdermal patch, adhesive or gel, a buccal patch, adhesive or gel, or a spray or an aerosol for administration to the lungs.

21. A composition according to anyone of claims 15 - 18 for transdermal administration behind the ear of a human body.

22. Method for manufacturing a nicotine-containing composition comprising mixing nicotine and one or more polar lipids and one or more anionic surfactants in sufficient amounts to form a liquid crystalline phase or a precursor or offspring thereof when placed in a polar solvent.

23. Method according to claim 22 wherein the one or more polar lipids are monoglycerides.

24. Method according to anyone of claims 22 or 23 wherein the one or more anionic surfactants are fatty acids.

25. Method according to claim 24 wherein the one or more fatty acid(s) is/are chosen from the group consisting of stearic acid, palmitic acid, oleic acid, linoleic acid, linolenic acid and arachidonic acid.

26. Method according to claim 25 wherein the fatty acid is oleic acid.

27. Method according to anyone of claims 23 - 26 wherein the monoglyceride is monoolein.

28. Method according to anyone of claims 23 - 26 wherein the monoglyceride is monolinolein.

29. Method according to anyone of claims 22 - 28 wherein with the other ingredients is mixed a local analgesic or a mixture of local analgesics.

30. Method according to claim 29 wherein the local analgesic(s) is/are selected from bensyl alcohol, benzocaine, chlorbutanol, chloroprocaine, clove, eugenol, lidocain, lidocain hydrochloride, mepivacaine, phenol, prilocaine, procaine, tetracaine, tetracaine hydrochloride and salicyl alcohol, or combinations thereof.

31. Method according to claim 30 wherein the local analgesic is bensocaine.

32. Method according to claim 30 wherein the local analgesic is bensyl alcohol.

33. Method of achieving cessation of using tobacco or of obtaining replacement of using tobacco whereby a composition according to anyone of claims 1 - 21 is administered to a human being in need thereof.

34. Use of a composition according to anyone of claims 1 - 21 for the manufacture of a medicament for treating Alzheimer's disease, Parkinson's disease or ulcerative colitis in a human being in need thereof.

## Patentansprüche

1. Zusammensetzung, umfassend Nikotin, ein oder mehrere polare Lipide und ein oder mehrere anionische Tenside in ausreichenden Mengen zur Bildung einer flüssigkristallinen Phase oder eines Vorläufers oder Abkömmlings davon, wenn sie in ein polares Lösungsmittel gegeben wird.

2. Zusammensetzung nach Anspruch 1, wobei das polare Lösungsmittel eine wässrige Lösung, Glycerol oder Propylenglycerol oder eine Mischung davon ist.

3. Zusammensetzung nach mindestens einem der Ansprüche 1 oder 2, wobei die flüssikristalline Phase oder ein Vorläufer oder Abkömmling davon irgendeiner der unten genannten Typen ist:
kubische, flüssigkristalline Phasen vom Typ I oder Typ II;
hexaganole, flüssigkristalline Phasen vom Typ I oder Typ II;
intermediäre flüssigkristalline Phasen vom Typ I oder Typ II und lamellare Phasen, in allen Fällen ohne Rücksicht auf die Raumgruppenanordnung, und Vorläufer von oder Abkömmling zu den flüssigen kristallinen Phasen, einschließlich jeglicher unterschiedlichen Phase oder Mischung davon, wie nach oder während der Anwendung der Formulierung durch die so induzierte Veränderung oder Veränderungen angenommen, die durch Veränderungen einer physikalischen oder einer chemischen Natur mit einer Wirkung auf eine oder mehrere der das System definierenden Zustandvariablen erfolgen;
feste Phasen;
Lösungsphasen vom Mizellar-Typ I oder -Typ II;
Lösungsphase vom Zweischicht-Typ einschließlich der Schwammphase (L₃-Phase), der L₂-Phase, Mikroemulsionen und echten Lösungen.

4. Zusammensetzung nach mindestens einem der Ansprüche 1 - 3, wobei das eine oder mehrere polare Lipide Monoglyceride sind.

5. Zusammensetzung nach mindestens einem der Ansprüche 1 - 4, wobei das eine oder mehrere anionische Tenside Fettsäuren sind.

6. Zusammensetzung nach Anspruch 5, wobei die eine oder mehrere Fettsäure(n) gewählt ist/sind aus der Gruppe bestehend aus Stearinsäure, Palmitinsäure, Oleinsäure, Linolsäure, Linolensäure und Arachidonsäure.

7. Zusammensetzung nach Anspruch 6, wobei die Fettsäure Oleinsäure ist.

8. Zusammensetzung nach mindestens einem der Ansprüche 1 - 7, weiterhin umfassend ein Lokalanalgetikum oder eine Mischung von Lokalanalgetika.

9. Zusammensetzung nach Anspruch 8, wobei das/die Analgetikum(a) gewählt ist/sind aus Bensylalkohol, Benzocain, Chlorbutanol, Chlorprocain, Nelke, Eugenol, Lidocain, Lidocainhydrochlorid, Mepivacain, Phenol, Prilocain, Procain, Tetracain, Tetracainhydrochlorid und Salicylalkohol oder Kombinationen davon.

10. Zusammensetzung nach Anspruch 9, wobei das Lokalanalgetikum Bensocain ist.

11. Zusammensetzung nach Anspruch 9, wobei das Lokalanalgetikum Bensylalkohol ist.

12. Zusammensetzung nach mindestens einem der Ansprüche 3 - 11, wobei das eine oder mehrere Monoglycerid(e) gewählt ist/sind aus der Gruppe bestehend aus Glycerolestern von Palmitoleinsäure, Oleinsäure, Linolsäure, Linolensäure und Arachidonsäure.

13. Zusammensetzung nach mindestens einem der Ansprüche 3 - 11, wobei das Monoglycerid Monoolein ist.

14. Zusammensetzung nach mindestens einem der Ansprüche 3 - 11, wobei das Monoglycerid Monolinolein ist.

15. Zusammensetzung nach mindestens einem der vorhergehenden Ansprüche, wahlweise weiter umfassend pharmazeutisch annehmbare Excipientien, für die Verwendung als Pharmazeutikum.

16. Zusammensetzung nach Anspruch 15, wobei die optionalen, pharmazeutisch annehmbaren Excipientien gewählt sind aus der Gruppe bestehend aus Geschmacks- bzw. Aromamitteln, Süßstoffen, Puffermitteln und Konservierungsmitteln.

17. Zusammensetzung nach mindestens einem der Ansprüche 15 oder 16 für die Verwendung bei der Tabaksubstitution, als Tabakersatz oder zur Einstellung des Rauchens.

18. Zusammensetzung nach mindestens einem der Ansprüche 15 oder 16 zur Behandlung von Alzheimer, Parkinson oder Colitis ulcerosa.

19. Zusammensetzung nach mindestens einem der Ansprüche 15 - 18 für die nasale, buccale, transdermale, mucosale oder pulmonale Verabreichung.

20. Zusammensetzung nach mindestens einem der Ansprüche 15 - 18 zur Verabreichung mittels eines Nasensprays oder -gels, eines buccalen Sprays, eines Kaugummis, einer Tablette, einer Pastille, eines transdermalen Pflasters, eines Klebstoffs oder Gels, eines buccalen Pflasters, eines Klebstoffs oder Gels, oder eines Sprays oder eines Aerosols für die Verabreichung an die Lungen.

21. Zusammensetzung nach mindestens einem der Ansprüche 15 - 18 für die transdermale Verabreichung hinter dem Ohr eines menschlichen Körpers.

22. Verfahren zur Herstellung einer nikotinhaltigen Zusammensetzung, umfassend das Mischen von Nikotin und einem oder mehreren polaren Lipiden und einem oder mehreren anionischen Tensiden in ausreichenden Mengen zur Bildung einer flüssigkristallinen Phase oder eines Vorläufers oder Abkömmlings davon, wenn sie in ein polares Lösungsmittel gegeben wird.

23. Verfahren nach Anspruch 22, wobei das eine oder mehrere polare Lipide Monoglyceride sind.

24. Verfahren nach mindestens einem der Ansprüche 22 oder 23, wobei das eine oder mehrere anionische Tenside Fettsäuren sind.

25. Verfahren nach Anspruch 24, wobei die eine oder mehrere Fettsäure(n) gewählt ist/sind aus der Gruppe bestehend aus Stearinsäure, Palmitinsäure, Oleinsäure, Linolsäure, Linolensäure und Arachidonsäure.

26. Verfahren nach Anspruch 25, wobei die Fettsäure Oleinsäure ist.

27. Verfahren nach mindestens einem der Ansprüche 23 - 26, wobei das Monoglycerid Monoolein ist.

28. Verfahren nach mindestens einem der Ansprüche 23 - 26, wobei das Monoglycerid Monolinolein ist.

29. Verfahren nach mindestens einem der Ansprüche 22 - 28, wobei mit den anderen Bestandteilen ein Lokalanalgetikum oder eine Mischung von Analgetika vermischt ist.

30. Verfahren nach Anspruch 29, wobei das/die Lokalanalgetikum(a) gewählt ist/sind aus Bensylalkohol, Benzocain, Chlorbutanol, Chlorprocain, Nelke, Eugenol, Lidocain, Lidocainhydrochlorid, Mepivacain, Phenol, Prilocain, Procain, Tetracain, Tetracainhydrochlorid und Salicylalkohol oder Kombinationen davon.

31. Verfahren nach Anspruch 30, wobei das Lokalanalgetikum Bensocain ist.

32. Verfahren nach Anspruch 30, wobei das Lokalanalgetikum Bensylalkohol ist.

33. Verfahren zur Erreichung der Einstellung des Tabakkonsums oder zum Erhalt eines Ersatzes für den Tabakkonsum, wodurch eine Zusammensetzung gemäß mindestens einem der Ansprüche 1 - 21 an einen Menschen, welcher daran Bedarf hat, verabreicht wird.

34. Anwendung einer Zusammensetzung nach mindestens einem der Ansprüche 1 - 21 für die Herstellung eines Medikaments zur Behandlung von Alzheimer, Parkinson oder Colitis ulcerosa bei einem Menschen, welcher daran Bedarf hat.

## Revendications

1. Composition contenant de la nicotine, un ou plusieurs lipides polaires et un ou plusieurs agents tensioactifs anioniques en des quantités suffisantes pour former une phase cristalline liquide ou un de ses précurseurs ou un de ses dérivés lorsqu'elle est placée dans un solvant polaire.

2. Composition selon la revendication 1, dans laquelle le solvant polaire est une solution aqueuse, du glycérol ou du propylèneglycol, ou un de leurs mélanges.

3. Composition selon l'une quelconque des revendications 1 ou 2, dans laquelle la phase cristalline liquide ou son précurseur ou son dérivé est de l'un quelconque des types ci-dessous:
phases cristallines liquides cubiques de type I ou de type II,
phases cristallines liquides hexagonales de type I ou de type II,
phases cristallines liquides intermédiaires de type I ou de type II, et phases lamellaires, dans tous les cas quel que soit l'arrangement des groupes spatiaux, et les précurseurs ou les dérivés de ladite phase cristalline liquide, comprenant une phase différente quelconque ou un mélange de telles phases, tels qu'adoptés après ou pendant l'application de la formulation par le ou les changements ainsi induits se produisant par l'intermédiaire de changements de nature physique et/ou chimique ayant un effet sur l'une ou plusieurs des variables d'état définissant le système,
phases solides,
phases de solution de type micellaire I ou II,
phase de solution de type bicouche comprenant la phase spongieuse (phase L₃), la phase L₂, des micro-émulsions et des solutions authentiques.

4. Composition selon l'une quelconque des revendications 1 à 3, dans laquelle le ou les lipides polaires sont des monoglycérides.

5. Composition selon l'une quelconque des revendications 1 à 4, dans laquelle le ou les agents tensioactifs anioniques sont des acides gras.

6. Composition selon la revendication 5, dans laquelle le ou les acides gras sont choisis dans le groupe constitué par l'acide stéarique, l'acide palmitique, l'acide oléique, l'acide linoléique, l'acide linolénique et l'acide arachidonique.

7. Composition selon la revendication 6, dans laquelle l'acide gras est l'acide oléique.

8. Composition selon l'une quelconque des revendications 1 à 7, contenant en outre un analgésique local ou un mélange d'analgésiques locaux.

9. Composition selon la revendication 8, dans laquelle l'analgésique local ou les analgésiques locaux sont choisis parmi l'alcool benzylique, la benzocaïne, le chlorbutanol, la chloroprocaïne, le clou de girofle, l'eugénol, la lidocaïne, le chlorhydrate de lidocaïne, la mépivacaïne, le phénol, la prilocaïne, la procaïne, la tétracaïne, le chlorhydrate de tétracaïne et l'alcool salicylique, ou leurs combinaisons.

10. Composition selon la revendication 9, dans laquelle l'analgésique local est la benzocaïne.

11. Composition selon la revendication 9, dans laquelle l'analgésique local est l'alcool benzylique.

12. Composition selon l'une quelconque des revendications 3 à 11, dans laquelle le ou les monoglycérides sont choisis dans le groupe constitué par des esters glycériques de l'acide palmitoléique, de l'acide oléique, de l'acide linoléique, de l'acide linolénique et de l'acide arachidonique.

13. Composition selon l'une quelconque des revendications 3 à 11, dans laquelle le monoglycéride est la monooléine.

14. Composition selon l'une quelconque des revendications 3 à 11, dans laquelle le monoglycéride est la monolinoléine.

15. Composition selon l'une quelconque des revendications précédentes, contenant éventuellement en outre des excipients pharmaceutiquement acceptables, à utiliser comme médicament.

16. Composition selon la revendication 15, dans laquelle les excipients pharmaceutiquement acceptables éventuels sont choisis dans le groupe constitué par des agents aromatisants, des édulcorants, des tampons et des conservateurs.

17. Composition selon l'une quelconque des revendications 15 ou 16, à utiliser pour substituer le tabac, remplacer le tabac ou arrêter de fumer.

18. Composition selon l'une quelconque des revendications 15 ou 16, pour le traitement de la maladie d'Alzheimer, de la maladie de Parkinson ou de la rectocolite hémorragique.

19. Composition selon l'une quelconque des revendications 15 à 18, destinée à l'administration nasale, buccale, transdermique, mucosale ou pulmonaire.

20. Composition selon l'une quelconque des revendications 15 à 18, destinée à l'administration par l'intermédiaire de pulvérisations nasales ou d'un gel nasal, de pulvérisations buccales, de gommes à mâcher, de comprimés, de pastilles, de timbres, d'adhésifs ou de gels transdermiques, de timbres, d'adhésifs ou de gels buccaux, ou de pulvérisations ou d'aérosols pour l'administration dans les poumons.

21. Composition selon l'une quelconque des revendications 15 à 18, pour l'administration transdermique derrière l'oreille d'un corps humain.

22. Procédé de fabrication d'une composition contenant de la nicotine, comprenant le mélange de nicotine avec un ou plusieurs lipides polaires et un ou plusieurs agents tensioactifs anioniques en des quantités suffisantes pour que se forme une phase cristalline liquide ou un de ses précurseurs ou un de ses dérivés lors de l'introduction dans un solvant polaire.

23. Procédé selon la revendication 22, dans lequel le ou les lipides polaires sont des monoglycérides.

24. Procédé selon l'une quelconque des revendications 22 ou 23, dans lequel le ou les agents tensioactifs anioniques sont des acides gras.

25. Procédé selon la revendication 24, dans lequel le ou les acides gras sont choisis dans le groupe constitué par l'acide stéarique, l'acides palmitique, l'acide oléique, l'acide linoléique, l'acide linolénique et l'acide arachidonique.

26. Procédé selon la revendication 25, dans lequel l'acide gras est l'acide oléique.

27. Procédé selon l'une quelconque des revendications 23 à 26, dans lequel le monoglycéride est la monooléine.

28. Procédé selon l'une quelconque des revendications 23 à 26, dans lequel le monoglycéride est la monolinoléine.

29. Procédé selon l'une quelconque des revendications 22 à 28, dans lequel on mélange un analgésique local ou un mélange d'analgésiques locaux avec les autres ingrédients.

30. Procédé selon la revendication 29, dans lequel l'analgésique local ou les analgésiques locaux sont choisis parmi l'alcool benzylique, la benzocaïne, le chlorbutanol, la chloroprocaïne, le clou de girofle, l'eugénol, la lidocaïne, le chlorhydrate de lidocaïne, la mépivacaïne, le phénol, la prilocaïne, la procaïne, la tétracaïne, le chlorhydrate de tétracaïne et l'alcool salicylique, ou leurs combinaisons.

31. Procédé selon la revendication 30, dans lequel l'analgésique local est la benzocaïne.

32. Procédé selon la revendication 30, dans lequel l'analgésique local est l'alcool benzylique.

33. Procédé pour cesser l'utilisation du tabac ou obtenir le remplacement de l'utilisation du tabac, selon lequel on administre une composition selon l'une quelconque des revendications 1 à 21 à un être humain qui en a besoin.

34. Utilisation d'une composition selon l'une quelconque des revendications 1 à 21 pour la fabrication d'un médicament destiné au traitement de la maladie d'Alzheimer, de la maladie de Parkinson ou de la rectocolite hémorragique chez un patient qui en a besoin.
